# EUROPEAN PATENT SPECIFICATION

(11) **EP 1 854 454 B1**
(45) Date of publication and mention of the grant of the patent: **06.11.2013**
(21) Application number: 07115060.1
(22) Date of filing: 16.01.2002
(51) Int. Cl.: A61K 9/16, A61K 31/4184

(54) **Method for the preparation of amorphous telmisartan**
Verfahren zur Herstellung von amorphen Telmisartan
Procédé de préparation du telmisartan amorphe

(43) Date of publication of application: 14.11.2007
(62) Divisional of application: 02708290.8
(73) Proprietor: Boehringer Ingelheim Pharma GmbH & Co. KG, 55216 Ingelheim am Rhein (DE)
(72) Inventor: Friedl, Thomas, Dr., 88416, OCHSENHAUSEN (DE); Schepky, Gottfried, Dr., 79312, EMMENDINGEN (DE)
(74) Representative: Simon, Elke Anna Maria

(56) References cited:
- WO-A-00/27397
- WO-A-00/43370

## Description

### Field of invention

The present invention describes a bilayer pharmaceutical tablet formulation comprising the angiotensin II receptor antagonist telmisartan and discloses method for the preparation of amorphous telmisartan.

### Background of the invention

INN Telmisartan is an angiotensin II receptor antagonist developed for the treatment of hypertension and other medical indications as disclosed in EP-A-502314.

Its chemical name is 4'-[2-n-propyl-4-methyl-6-(1-methylbenzimidazol-2-yl)-benzimidazol-1-ylmethyl]-biphenyl-2-carboxylic acid having the following structure:

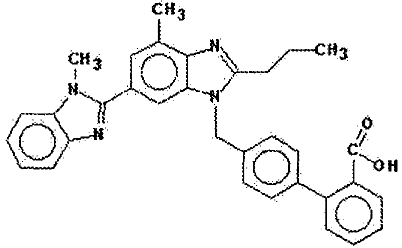

Telmisartan is generally manufactured and supplied in the free acid form. It is characterized by its very poor solubility in aqueous systems at the physiological pH range of the gastro-intestinal tract of between pH 1 to 7. As disclosed in WO 00/43370, and WO 00/27397 telmisartan can exist in two polymorphic forms having different melting points and in an amorphous form. Under the influence of heat and humidity, the lower melting polymorph B transforms irreversibly into the higher melting polymorph A.

### Summary of the invention

In accordance with the present invention, a drug comprising telmisartan is prepared as a tablet layer containing telmisartan in substantially amorphous form in a dissolving tablet matrix.

The bilayer tablet provides a largely pH-independent dissolution of the poorly water-soluble telmisartan, thereby facilitating dissolution of the drug at a physiological pH level.

The present invention provides a method according to claim 1, for the preparation of telmisartan in at least 90% amorphous form comprising
a) preparing an aqueous solution of telmisartan, at least one basic agent and, optionally, a solubilizer and/or a crystallization retarder; and
b) spray-drying said aqueous solution to obtain a spray-dried granulate.

### Definitions

As used herein, the term "substantially amorphous" refers to a product comprising amorphous constituents in a proportion of at least 90%, preferably at least 95%, as determined by X-ray powder diffraction measurement.

The term "dissolving tablet matrix" refers to a pharmaceutical tablet base formulation having immediate release (fast dissolution) characteristics that readily dissolves in a physiological aqueous medium.

### Description of the preferred embodiments

A bilayer tablet can comprise telmisartan in substantially amorphous form in a dissolving tablet matrix.

The active ingredient telmisartan is generally supplied in its free acid form, although pharmaceutically acceptable salts may also be used. Since telmisartan is dissolved and transformed into a substantially amorphous form, its initial crystal morphology and particle size are of little importance for the physical and biopharmaceutical properties of the formulation obtained. It is however preferred to remove agglomerates from the starting material, e.g. by sieving, in order to facilitate wetting and dissolution during further processing.

Substantially amorphous telmisartan is prepared by the specific spray-drying method described hereinafter.

A tablet layer is described that contains telmisartan in substantially amorphous form dispersed in a dissolving tablet matrix having immediate release (fast dissolution) characteristics. The dissolving tablet matrix may have acidic, neutral or basic properties.

In a basic tablet matrix the dissolving matrix comprises a basic agent, a water-soluble diluent and, optionally, other excipients and adjuvants.

Specific examples of suitable basic agents are alkali metal hydroxides such as NaOH and KOH; basic amino acids such as arginine and lysine; and meglumine (N-methyl-D-glucamin), NaOH and meglumine being preferred.

Specific examples of suitable water-soluble diluents are carbohydrates such as monosaccharides like glucose; oligosaccharides like sucrose, anhydrous lactose and lactose monohydrate; and sugar alcohols like sorbitol, mannitol, dulcitol, ribitol and xylitol. Sorbitol is a preferred diluent.

The other excipients and/or adjuvants are, for instance, selected from binders. carriers, fillers, lubricants, flow control agents, crystallization retarders such as Povidone etc., solubilizers, coloring agents, pH control agents, surfactants and emulsifiers such as a Pluronic (poloxamer), polyethylene glycols, sodium carboxymethyl cellulose, polyethoxylated and hydrogenated castor oil, etc. The excipients and/or adjuvants for the first tablet layer composition are preferably chosen such that a non-acidic, fast dissolving tablet matrix is obtained.

The tablet layer composition generally comprises 3 to 50 wt.%, preferably 5 to 35 wt.%, of active ingredient; 0.25 to 20 wt.%, preferably 0.40 to 15 wt.%, of basic agent; and 30 to 95 wt.%, preferably 60 to 80 wt.% of water-soluble diluent.

Other (optional) constituents may, for instance, be chosen from one or more of the following excipients and/or adjuvants in the amounts indicated:
10 to 30 wt.%, preferably 15 to 25 wt.%, of binders, carriers and fillers, thereby replacing the water-soluble diluent;
0.1 to 5 wt.%, preferably 0.5 to 3 wt.%, of lubricants;
0.1 to 5 wt.%, preferably 0.3 to 2 wt.%, of flow control agents;
1 to 10 wt.%, preferably 2 to 8 wt.%, of crystallization retarders;
1 to 10 wt.%, preferably 2 to 8 wt.%, of solubilizers;
0.05 to 1.5 wt.%, preferably 0.1 to 0.8 wt.%, of coloring agents;
0.5 to 10 wt.%, preferably 2 to 8 wt.%, of pH control agents;
0.01 to 5 wt.%, preferably 0.05 to 1 wt.%, of surfactants and emulsifiers.

For preparing a bilayer tablet first and second tablet layer compositions may be compressed in the usual manner in a bilayer tablet press, e.g. a high-speed rotary press in a bilayer tableting mode. However, care should be taken not to employ an excessive compression force for the first tablet layer comprising telmisartan. Preferably, the ratio of the compression force applied during compression of the first tablet layer to the compression force applied during compression of both the first and second tablet layers is in the range of from 1:10 to 1:2. For instance, the first tablet layer may be compressed at moderate force of 4 to 8 kN, whereas the main compression of first plus second layer is performed at a force of 10 to 20 kN.

During bilayer tablet compression adequate bond formation between the two layers is achieved by virtue of distance attraction forces (intermolecular forces) and mechanical interlocking between the particles.

In accordance with the present invention, at least 70% and typically at least 90% of the amorphous telmisartan are dissolved after 30 min.

For optimum dissolution and drug release properties, the method according to the present invention comprises
a) preparing an aqueous solution of telmisartan, at least one basic agent and, optionally, a solubilizer and/or a crystallization retarder;
b) spray-drying said aqueous solution to obtain a spray-dried granulate; and
e) optionally, adding other excipients and/or adjuvants in any of steps a) to d).

In a preferred embodiment of this method, an aqueous alkaline solution of telmisartan is prepared by dissolving telmisartan in purified water with the help of one or more basic agents like sodium hydroxide and meglumine. Optionally, a solubilizer and/or a recrystallization retarder may be added. The dry matter content of the starting aqueous solution is generally 10 to 40 wt.%, preferably 20 to 30 wt.%.

The aqueous solution is then spray-dried at room temperature or preferably at increased temperatures of, for instance, between 50 and 100°C in a co-current or countercurrent spray-drier at a spray pressure of 1 to 4 bar. Generally speaking, the spray-drying conditions are preferably chosen in such a manner that a spray-dried granulate having a residual humidity of ≤ 5 wt.%, preferably ≤ 3.5 wt.%, is obtained in the separation cyclone. To that end, the outlet air temperature of the spray-drier is preferably kept at a value of between about 80 and 90°C while the other process parameters such as spray pressure, spraying rate, inlet air temperature, etc. are adjusted accordingly.

The spray-dried granulate obtained is a fine powder having the following particle size distribution:
d₁₀: ≤ 20 µm, preferably ≤ 10 µm
d₅₀ : ≤ 80 µm, preferably 20 to 55 µm
d₉₀ : ≤ 350 µm, preferably 50 to 150 µm

After spray-drying, the active ingredient (telmisartan) as well as the excipients contained in the spray-dried granulate are in a substantially amorphous state with no crystallinity being detectable. From a physical point of view, the spray-dried granulate is a solidified solution or glass having a glass transition temperature Tg of preferably > 50°C, more preferably > 80°C.

Based on 100 parts by weight of active ingredient (telmisartan), the spray-dried granulate preferably contains 5 to 200 parts by weight of basic agent and, optionally, solubilizer and/or crystallization retarder.

Water-soluble diluent can be employed in an amount of 30 to 95 wt.%, preferably 60 to 80 wt.%, based on the weight of the tablet layer composition.

Lubricant can be added in an amount of 0.1 to 5 wt.%, preferably 0.3 to 2 wt.%, based on the weight of the tablet layer composition.

Mixing is carried out in two stages, i.e. in a first mixing step the spray-dried granulate and the diluent are admixed using , e.g., a high-shear mixer or a free-fall blender, and in a second mixing step the lubricant is blended with the premix, preferably also Under conditions of high shear. The method is however not limited to these mixing procedures and, generally, alternative mixing procedures may be employed.

For production of the bilayer tablet first and second tablet layercompositions can be compressed in a bilayer tablet press, e.g. a rotary press in the bilayer tableting mode, in the manner described above. In order to avoid any cross-contamination between the first and second tablet layers (which could lead to decomposition of HTCZ), any granulate residues have to be carefully removed during tableting by intense suction of the die table within the tableting chamber.

In order to further illustrate the present invention, the following non-limiting examples are given.

### Example 1

| | Constituents | mg / 1.684 mg SD granulate | volatile constituent | kg / batch |
|---|---|---|---|---|
| (01) | Telmisartan | 1.000 | | 45.000 |
| (02) | Sodium hydroxide | 0.084 | | 3.780 |
| (03) | Povidone K 25 | 0.300 | | 13.500 |
| (04) | Meglumine | 0.300 | | 13.500 |
| (05) | Purified water | | 5.000 | (225.000) |
| | | 1.684 | 5.000 | 75.780 |

### Manufacturing:

### 1. Spray solution

225.000 kg of purified water are measured into a suitable stainless steel vessel at a temperature of between 20-40°C. In sequence, 3.780 of kg sodium hydroxide, 45.000 kg of telmisartan (mixture of polymorph A and B), 13.500 kg of Povidone K 25 and 13.500 kg of meglumine are dissolved in the purified water under intensive stirring until a virtually clear, slightly yellowish, alkaline solution is obtained.

### 2. Spray drying

The solution is sprayed into a suitable spray dryer, e.g. a Niro P 6.3 equipped with Schlick atomizing nozzles of 1.0 mm diameter, with a flow-through heating coil connected upstream of the dryer, and dried to give a white to off-white fine granulate. The spray mode is counter-current at a spray-pressure of about 3000 hPa (3 bar) an inlet air temperature of about 125°C and a spray rate of about 11 kg/h, thus resulting in an outlet air temperature of about 85°C. The temperature of the flow through heating coil water bath is set at a temperature of about 80°C.

### 3. Protective Screening

The dry granulate powder is screened through a screen of 0.5 mm mesh size, e.g. using a Vibra Sieve machine.

The resulting amorphous telmisartan spray-dried granulate may be further processed to telmisartan mono-tablets or the first layer of the said bilayer tablet composition.

### Reference Example 2

| | Constituents | mg/tablet 1st layer | mg/SD granulate | mg/tablet 2nd layer |
|---|---|---|---|---|
| (01) | Telmisartan SD granulate | 67.360 | | |
| | consisting of (02) to (06): | | | |
| (02) | Telmisartan | | 40.000 | |
| (03) | Sodium hydroxide | | 3.360 | |
| (04) | Polyvidone (Kollidon 25) | | 12.000 | |
| (05) | Meglumine | | 12.000 | |
| (06) | Purified water | | 264.000* | |
| (07) | Sorbitol P/6 | 168.640 | | |
| (08) | Magnesium stearate, screened | 4.000 | | 1.000 |
| (09) | Hydrochlorothiazide | | | 12.500 |
| (10) | Microcrystalline cellulose (Avicel PH 101) | | | 64.000 |
| (11) | Red iron oxide | | | 0.330 |
| (12) | Sodium starch glycolate | | | 4.000 |
| (13) | Lactose monohydrate fine, screened | | | 112.170 |
| (14) | Maize starch, dried at 45 °C | | | 6.000 |
| | | 240.000 | 67.360 | 200.000 |

| | | | | |
|---|---|---|---|---|
| * 200 mg in SD granulate, 64 mg in granulation liquid of HCTZ granulate | | | | |

### Manufacturing:

### 1. Final blend A

168.640 kg of sorbitol are mixed with 67.360 kg of telmisartan spray dried granulate in a suitable high shear mixer, e.g. Diosna P 600, for 4 minutes using both impeller and chopper. Next 4.0 kg of magnesium stearate are added to the resulting pre-mix and admixed in the high shear mixer for further 30 seconds.

### 2. Final blend B

9.000 kg of purified water of about 70°C are transfered to a suitable mixing vessel, 6.000 kg of maize starch, dried at 45°C, are suspended in the water. This suspension is stirred into 55.000 kg of purified water of about 90°C using e.g. an Ekato stirrer.

Next, 112.170 kg of lactose monohydrate, 12.500 kg of hydrochlorothiazide, 64.000 kg of microcrystalline cellulose (Avicel PH 101), 0.330 kg of red iron oxide and 4.000 kg of sodium starch glycolate are mixed in a suitable high shear granulator, e.g. Diosna P 600, until homogeneous, and moistened with 70.000 kg of the above-prepared aqueous granulating liquid.

### Process parameters for wet granulation:

| Process step | Duration (min) | Impeller (setting) | Chopper (setting) |
|---|---|---|---|
| Pre-mixing | 3 | 1 | 1 |
| Moistening | 2 | 1 | 1 |
| Wet mixing | 4 | 2 | 2 |
| Emptying | About 0.5 | 1 | 0 |

After moistening, the resulting wet granulate is dried in a suitable fluid bed dryer, e.g. Glatt WSG 120 at an inlet air temperature of 100°C, an inlet air flow of 2000-3000 m³/h until a product temperature of about 55°C is reached.

The dry granulate is screened to reduce the particle size using a suitable screening machine, e.g. a Comil screen machine equipped with a rasp screen of 2 mm mesh size. Finally 1.000 kg of prescreened magnesium stearate are admixed to the screened granulate material and mixed in a suitable tumbling mixer, e.g. a Lermer rotating spike mixer, for 100 revolutions at a speed of 8-10 rpm.

### 3. Bilayer tablet compression

Using a suitable rotary tablet press, 240 kg of the final blend (A) and 200 kg of the final blend (B) are compressed into bilayer tablets. The target weight for the first layer is 240 mg, the target weight for the second layer is 200 mg.

### Process parameters for tableting:

| Tablet press | Fette 3090 | |
|---|---|---|
| Tabletting speed | 100.000 (80.000 - 120.000) tabl/h | |
| Stirrer blade speed: | 1st layer about 30 rpm | 2nd layer about 75 rpm |
| Compression force | 5 (4 - 6) kN | 12 (10 - 14) kN |

As a rule, the tablet hardness is adjusted by variation of the main compression force of the second layer.

The resulting bilayer tablets have the following characteristics:

| | |
|---|---|
| Shape / diameter | oval, both faces convex / 14 x 6.8 mm |
| Colour | first layer: white to off-white |
| | second layer: red |
| Weight | 440 mg (total) |
| | 240 mg (layer 1: with telmisartan) |
| | 200 mg (layer 2: with hydrochlorothiazide) |
| Thickness | about 5.2 mm |
| Hardness | about 120 N |
| Disintegration time | NMT 15 min (total) |

### Reference Example 3

| | Constituents | mg/tablet 1st layer | mg/SD qranulate | mg/tablet 2nd layer |
|---|---|---|---|---|
| (01) | Telmisartan SD granulate | 67.360 | | |
| | consisting of (02) to (06): | | | |
| (02) | Telmisartan | | 40.000 | |
| (03) | Sodium hydroxide | | 3.360 | |
| (04) | Polyvidone (Kollidon 25) | | 12.000 | |
| (05) | Meglumine | | 12.000 | |
| (06) | Purified water | | (200.000) | |
| (07) | Sorbitol P/6 | 168.640 | | |
| (08) | Magnesium stearate, screened | 4.000 | | 1.000 |
| (09) | Hydrochlorothiazide | | | 25.000 |
| (10) | Microcrystalline cellulose (Avicel PH 101) | | | 64.000 |
| (11) | Yellow iron oxide | | | 0.330 |
| (12) | Sodium starch glycolate | | | 4.000 |
| (13) | Lactose monohydrate fine, screened | | | 105.67 |
| | | 240.000 | 67.360 | 200.000 |

### Manufacturing:

Manufacturing is carried out as in Example 2. Instead of the wet granulation process described in Example 2, the second layer composition is manufactured by dry mixing of (09) to (13) in a suitable free fall blender, e.g. a 1 m³ container mixer, for 200 revolutions at a speed of 10 rpm. Then, (08) is admixed to the main mixture for further 50 revolutions in the container mixer. In order to achieve a homogenous distribution of the color pigment, an additional premix with yellow iron oxide and a portion of the microcrystalline cellulose, e.g. 2.000 kg, which is screened through an 0.8 mm mesh screen manually before transfer to the main mixture, may be performed. The resulting bilayer tablets display virtually the same physical characteristics as described in example 2, except for the color.

### Reference Example 4

### Composition of Telmisartan/Hydrochlorothiazide Bilayer Tablets (mg per tablet):

| **Ingredient** | | **40/12.5 mg** | **80/12.5 mg** |
|---|---|---|---|
| **Telmisartan layer** | | | |
| | Telmisartan | 40.000 | 80.000 |
| | Sodium hydroxide | 3.360 | 6.720 |
| | Povidone | 12.000 | 24.000 |
| | Meglumine | 12.000 | 24.000 |
| | Purified water* | (200.000) | (400.000) |
| | Sorbitol | 168.640 | 337.280 |
| | Magnesium stearate | 4.000 | 8.000 |
| Total telmisartan layer | | 240.000 | 480.000 |

| **Hydrochlorothiazide layer** | | | |
|---|---|---|---|
| | Hydrochlorothiazide | 12.500 | 12.500 |
| | Lactose monohydrate | 112.170 | 112.170 |
| | Microcrystalline Cellulose | 64.000 | 64.000 |
| | Corn starch | 6.000 | 6.000 |
| | Red iron oxide | 0.330 | 0.330 |
| | Sodium starch glycolate | 4.000 | 4.000 |
| | Purified water* | (64.000) | (64.000) |
| | Magnesium stearate | 1.000 | 1.000 |
| Total HCTZ layer | | 200.000 | 200.000 |
| **Total tablet weight** | | 440.000 | 680.000 |

| | | | |
|---|---|---|---|
| *Does not appear in final product | | | |

Also dislcosed are:
1. A bilayer pharmaceutical tablet comprising a first layer containing telmisartan in substantially amorphous form in a dissolving tablet matrix, and a second layer containing a diuretic in a disintegrating tablet matrix.
2. A bilayer pharmaceutical tablet as claimed in claim 1 wherein the diuretic is selected from at least one of hydrochlorothiazide, furosemide, chlorotalidone, piretanide and amiloride.
3. A bilayer pharmaceutical tablet as claimed in claim 2 wherein the diuretic is hydrochlorothiazide.
4. A bilayer pharmaceutical tablet as claimed in any one of claims 1-3 wherein the dissolving tablet matrix has immediate release characteristics.
5. A bilayer pharmaceutical tablet as claimed in any one of claims 1-4 wherein the dissolving tablet matrix comprises a basic agent, a water-soluble diluent and, optionally, other excipients and adjuvants.
6. A bilayer pharmaceutical tablet as claimed in claim 5 where the basic agent is selected from alkali metal hydroxides, basic amino acids and meglumine.
7. A bilayer pharmaceutical tablet as claimed in claims 5 or 6 wherein the water-soluble diluent is selected from carbohydrates such as monosaccharides like glucose; oligosaccharides like sucrose and lactose; and sugar alcohols like sorbitol, mannitol, dulcitol, ribitol and xylitol.
8. A bilayer pharmaceutical tablet as claimed in any one of claims 5-7 wherein the other excipients and adjuvants are selected from binders, carriers, fillers, lubricants, flow control agents, crystallization retarders, solubilizers, coloring agents, pH control agents, surfactants and emulsifiers.
9. A bilayer pharmaceutical tablet as claimed in any one of claims 1-8 wherein the first tablet layer has been produced by spray-drying an aqueous solution comprising telmisartan and a basic agent to obtain a spray-dried granulate, mixing said spray-dried granulate with a water-soluble diluent to obtain a premix, mixing said premix with a lubricant to obtain a final blend and compressing the final blend to form the first tablet layer.
10. A bilayer pharmaceutical tablet as claimed in any one of claims 1-10 wherein the disintegrating tablet matrix comprises a filler, a binder, a disintegrant and, optionally, other excipients and adjuvants.
11. A bilayer pharmaceutical tablet as claimed in claim 10 wherein the other excipients and adjuvants are selected from carriers, diluents, lubricants, flow control agents, solubilizers, coloring agents, pH control agents, surfactants and emulsifiers.
12. A bilayer pharmaceutical tablet as claimed in any one of claims 1-11 containing 10 to 160 mg, preferably 20 to 80 mg, of telmisartan and 6.25 to 50 mg, preferably 12.5 to 25 mg, of diuretic.
13. A bilayer pharmaceutical tablet as claimed in any one of claims 1-12 packaged in a moisture proof packaging material such as aluminium foil blister packs, or polypropylene tubes and HDPE bottles.
14. A method of producing a bilayer pharmaceutical tablet comprising the steps of:
   (i) providing a first tablet layer composition by
      a) preparing an aqueous solution of telmisartan, at least one basic agent and, optionally, a solubilizer and/or a crystallization retarder;
      b) spray-drying said aqueous solution to obtain a spray-dried granulate;
      c) mixing said spray-dried granulate with a water-soluble diluent to obtain a premix;
      d) mixing said premix with a lubricant to obtain a final blend for the first tablet layer;
      e) optionally, adding other excipients and/or adjuvants in any of steps a) to d);
   (ii) providing a second tablet layer composition by
      f) mixing and/or granulating a diuretic with the constituents of a disintegrating tablet matrix and, optionally, further excipients and/or adjuvants;
      g) admixing a lubricant to obtain a final blend for the second tablet layer;
   (iii) introducing the first or the second tablet layer composition into a tablet press;
   (iv) compressing said tablet layer composition to form a tablet layer;
   (v) introducing the other tablet layer composition into the tablet press; and
   (vi) compressing both tablet layer compositions to form a bilayer tablet.
15. A method as claimed in claim 14 wherein spray-drying in step b) is carried out under conditions so as to obtain a spray-dried granulate having a residual humidity of ≤ 5 wt.%, preferably ≤ 3.5 wt.%.
16. A method as claimed in claims 14 or 15 wherein spray-drying in step b) is carried out at an outlet air temperature of the spray-drier of between about 80 and 90°C.
17. A method as claimed in any one of claims 14-16 wherein mixing in any of steps c), d), f) and g) is carried out in a high shear mixer or a free-fall blender.
18. A method as claimed in any one of claims 14-17 wherein mixing in step f) is carried out under conditions of dry-mixing or, preferably, under wet granulation conditions.
19. A method as claimed in any one of claims 14-18 wherein the ratio of the compression force applied during compression of the first tablet layer to the compression force applied during compression of both the first and second tablet layers is in the range of from 1:10 to 1:2.

## Claims

1. A method for the preparation of telmisartan in at least 90% amorphous form as determined by X-ray powder diffraction measurement comprising
a) preparing an aqueous solution comprising telmisartan and at least one basic agent and
b) spray-drying said aqueous solution at room temperature or at increased temperatures of between 50°C and 100°C in a co-current or countercurrent spray-drier and at a spray pressure of 1 to 4 bar to obtain a spray-dried granulate having the following particle size distribution:
d₁₀ : ≤ 20 µm
d₅₀ : ≤ 80 µm
d₉₀ : ≤ 350 µm.

2. The method of claim 1, **characterized in that** based on 100 parts by weight of telmisartan the spray-dried granulate comprises 5 to 200 parts by weight of basic agent.

3. The method of claim 1, **characterized in that** telmisartan is dissolved in water with the help of one or more basic agent selected from the group consisting of an alkali metal hydroxide, a basic amino acid and meglumine.

4. The method of claim 2, **characterized in that** the basic agent is sodium hydroxide and/or meglumine.

5. The method of claim 1, **characterized in that** the aqueous solution of telmisartan additionally comprises a Pluronic poloxamer as solubilizer and/or Povidone as a crystallization retarder.

6. The method of claim 5, **characterized in that** the dry matter content of the starting aqueous solution is generally 10 to 40 wt.%.

7. The method of claim 1, **characterized in that** the aqueous solution is spray-dried in a counter-current spray-drier at a spray pressure of 3 bar with
• an inlet air temperature of 125°C,
• the flow through heating coil water bath set at a temperature of 80°C and
• a spray rate of 11 kg/h
• resulting in an outlet air temperature of about 85°C.

8. The method of claim 1, **characterized in that** a spray-dried granulate having a residual humidity of ≤ 5 wt.% is obtained in a separation cyclone.

9. The method of claim 8, **characterized in that** the outlet air temperature of the spray-drier is kept at about 80°C to 90°C.

10. The method of claim 1, **characterized in that** the spray-dried granulate is a solidified solution or glass having a glass transition temperature Tg of > 50°C.

11. The method of claim 1, **characterized in that** telmisartan and the excipients contained in the spray-dried granulate are in an amorphous state with no crystallinity being detectable.

## Patentansprüche

1. Verfahren zur Herstellung von Telmisartan in mindestens 90% amorpher Form, wie bestimmt durch röntgenpulverdiffraktometrische Messung, umfassend
a) Herstellen einer wässerigen Lösung, umfassend Telmisartan und mindestens ein basisches Mittel, und
b) Sprühtrocknen der wässerigen Lösung bei Raumtemperatur oder bei erhöhten Temperaturen zwischen 50°C und 100°C in einem Gleichstrom- oder Gegenstrom-Sprühtrockner und bei einem Sprühdruck von 1 bis 4 bar, um ein sprühgetrocknetes Granulat mit der nachfolgenden Partikelgrößenverteilung zu erhalten:
d₁₀: ≤ 20 µm
d₅₀: ≤ 80 µm
d₉₀: ≤ 350 µm.

2. Verfahren nach Anspruch 1, **dadurch gekennzeichnet, dass**, basierend auf 100 Gewichtsteilen Telmisarten, das sprühgetrocknete Granulat 5 bis 200 Gewichtsteile basisches Mittel aufweist.

3. Verfahren nach Anspruch 1, **dadurch gekennzeichnet, dass** das Telmisartan in Wasser mit Hilfe von ein oder mehreren basischen Mitteln, ausgewählt aus der Gruppe, bestehend aus einem Alkalimetallhydroxid, einer basischen Aminosäure und Meglumin, gelöst wird.

4. Verfahren nach Anspruch 2, **dadurch gekennzeichnet, dass** das basische Mittel Natriumhydroxid und/oder Meglumin darstellt.

5. Verfahren nach Anspruch 1, **dadurch gekennzeichnet, dass** die wässerige Lösung von Telmisartan zusätzlich ein Pluronic-Poloxamer als Lösungshilfsmittel und/oder Povidon als Kristallisationsverzögerer aufweist.

6. Verfahren nach Anspruch 5, **dadurch gekennzeichnet, dass** der Trockengehalt der wässerigen Ausgangslösung im Allgemeinen 10 bis 40 Gew.-% beträgt.

7. Verfahren nach Anspruch 1, **dadurch gekennzeichnet, dass** die wässerige Lösung in einem Gegenstrom-Sprühtrockner bei einem Sprühdruck von 3 bar sprühgetrocknet wird mit
- einer Lufteinlasstemperatur von 125°C,
- das Durchflusswasserbad mit Heizspirale eingestellt wird auf eine Temperatur von 80°C und
- einer Sprührate von 11 kg/h
- resultierend in einer Luftauslasstemperatur von etwa 85°C.

8. Verfahren nach Anspruch 1, **dadurch gekennzeichnet, dass** das sprühgetrocknete Granulat mit einen Restfeuchtigkeit von ≤ 5 Gew.-% in einem Fliehkraftabscheider bzw. Abscheidezyklon erhalten wird.

9. Verfahren nach Anspruch 8, **dadurch gekennzeichnet, dass** die Luftauslasstemperatur des Sprühtrockners bei etwa 80°C bis 90°C gehalten wird.

10. Verfahren nach Anspruch 1, **dadurch gekennzeichnet, dass** das sprühgetrocknete Granulat eine verfestigte Lösung oder ein Glas mit einer Glasübergangstemperatur Tg > 50°C darstellt.

11. Verfahren nach Anspruch 1, **dadurch gekennzeichnet, dass** das Telmisartan und die Hilfsstoffe, enthalten in dem sprühgetrockneten Granulat, sich in einem amorphen Zustand ohne nachweisbare Kristallinität befinden.

## Revendications

1. Procédé pour la préparation de telmisartan sous une forme amorphe à au moins 90 % comme déterminé par une mesure de diffraction des rayons X de poudre comprenant
a) la préparation d'une solution aqueuse comprenant du telmisartan et au moins un agent basique et
b) le séchage par pulvérisation de ladite solution aqueuse à la température ambiante ou à des températures accrues entre 50°C et 100°C dans un sécheur par pulvérisation à co-courant ou contre-courant et à une pression de pulvérisation de 1 à 4 bar pour obtenir des granulés séchés par pulvérisation ayant la distribution de tailles de particules suivante :
d₁₀ : ≤ 20 µm
d₅₀ : ≤ 80 µm
d₉₀ : ≤ 350 µm.

2. Procédé selon la revendication 1 **caractérisé en ce que**, sur la base de 100 parties en poids de telmisartan, les granulés séchés par pulvérisation comprennent 5 à 200 parties en poids d'agent basique.

3. Procédé selon la revendication 1 **caractérisé en ce que** le telmisartan est dissous dans l'eau à l'aide d'un ou plusieurs agents basiques choisis dans le groupe consistant en un hydroxyde de métal alcalin, un aminoacide basique et la méglumine.

4. Procédé selon la revendication 2 **caractérisé en ce que** l'agent basique est l'hydroxyde de sodium et/ou la méglumine.

5. Procédé selon la revendication 1 **caractérisé en ce que** la solution aqueuse de telmisartan comprend en outre un poloxamère Pluronic comme solubilisant et/ou de la Povidone comme retardateur de cristallisation.

6. Procédé selon la revendication 5 **caractérisé en ce que** la teneur en matière sèche de la solution aqueuse de départ est généralement 10 à 40 % en poids.

7. Procédé selon la revendication 1 **caractérisé en ce que** la solution aqueuse est séchée par pulvérisation dans un sécheur par pulvérisation à contre-courant à une pression de pulvérisation de 3 bar avec
• une température de l'air entrant de 125°C,
• le bain d'eau du serpentin réchauffeur à circulation réglé à une température de 80°C et
• un débit de pulvérisation de 11 kg/h
• conduisant à une température de l'air sortant d'environ 85°C.

8. Procédé selon la revendication 1 **caractérisé en ce que** des granulés séchés par pulvérisation ayant une humidité résiduelle de ≤ 5 % en poids sont obtenus dans un cyclone de séparation.

9. Procédé selon la revendication 8 **caractérisé en ce que** la température de l'air sortant du sécheur par pulvérisation est maintenue à environ 80°C à 90°C.

10. Procédé selon la revendication 1 **caractérisé en ce que** les granulés séchés par pulvérisation sont une solution solidifiée ou un verre ayant une température de transition vitreuse Tg de > 50°C.

11. Procédé selon la revendication 1 **caractérisé en ce que** le telmisartan et les excipients contenus dans les granulés séchés par pulvérisation sont dans un état amorphe sans cristallinité détectable.
